# EUROPÄISCHE PATENTSCHRIFT

(11) **EP 3 284 508 B1**
(45) Veröffentlichungstag und Bekanntmachung des Hinweises auf die Patenterteilung: **17.07.2019**
(21) Anmeldenummer: 16184297.6
(22) Anmeldetag: 16.08.2016
(51) Int. Cl.: A61N 1/05, A61B 90/98, A61N 1/02, A61N 1/372, A61N 1/37, G06K 19/077, A61B 5/00

(54) **IMPLANTAT UND VERFAHREN ZUR IDENTIFIKATION EINER ELEKTRODENLEITUNG**
IMPLANT AND METHOD FOR THE IDENTIFICATION OF AN ELECTRODE LEAD
IMPLANT ET PROCEDE D'IDENTIFICATION D'UNE LIGNE D'ELECTRODES

(43) Veröffentlichungstag der Anmeldung: 21.02.2018
(73) Patentinhaber: BIOTRONIK SE & Co. KG, 12359 Berlin (DE)
(72) Erfinder: Rump, Jens, 12049 Berlin (DE); Friedrich, Michael, 14532 Kleinmachnow (DE); Kolberg, Gernot, 12161 Berlin (DE); van Ooyen, André, 10717 Berlin (DE); Phung, Gia Ngoc, 14059 Berlin (DE); Neumann, Andreas, 12437 Berlin (DE); Frenzel, Timo, 12435 Berlin (DE); Weiss, Ingo, 12435 Berlin (DE); Moß, Christian, 10589 Berlin (DE); Busch, Ulrich, 10318 Berlin (DE); Skerl, Olaf, 18209 Bad Doberan (DE)
(74) Vertreter: Randoll, Sören

(56) Entgegenhaltungen:
- DE-A1-102008 040 867
- US-A1- 2007 203 547
- US-A1- 2014 330 347
- US-B2- 7 983 763

## Beschreibung

Die Erfindung betrifft eine Elektrodenleitung zur Verbindung mit einem Implantat mittels eines Steckers mit mindestens einem elektrischen Leiter und einem den mindestens einen elektrischen Leiter isolierenden Isolationsschlauch, ein Verfahren zur Identifikation einer Elektrodenleitung sowie ein Implantat mit einer Buchse zur Verbindung mit dem Stecker einer Elektrodenleitung.

Implantate (implantierbare Medizingeräte, IMD) wie Herzschrittmacher (Pacemaker), Defibrillatoren oder neurologische Geräte wie Himschrittmacher für tiefe Hirnstimulation (Deep Brain Stimulation), Rückenmarkstimulationsgeräte, TENS (Transcutaneous Electrical Nerve Stimulator), Geräte für muskuläre Stimulationstherapie, oder Diagnosegeräte, welche die chemischen Eigenschaften des Blutes des Patienten, anderer Körperteile oder andere Körpereigenschaften und -parameter untersuchen, verwenden häufig Elektrodenleitungen, die in den Körper des Patienten geführt werden und dort zumindest über den Zeitraum der Behandlung oder Messung verbleiben. Die Elektrodenleitungen sind mit dem Implantat elektrisch leitend verbunden.

Die Implantate umfassen üblicherweise ein körperverträgliches Gehäuse mit einer dazugehörigen elektronischen Schaltung und einer Energieversorgung, z. B. einer Batterie. Das Gehäuse besitzt mindestens eine Buchse, an der eine oder mehrere Elektrodenleitungen angeschlossen werden können, beispielsweise mittels eines Steckers. Eine Elektrodenleitung dient der Übertragung der elektrischen Energie bzw. von Daten vom Gehäuse zu dem behandelten oder untersuchten Körperteil und umgekehrt.

Im Rahmen der vorliegenden Erfindung wird unter dem Begriff "Elektrodenleitung" eine Leitung mit mindestens einem elektrischen Leiter oder mehreren elektrischen Leitern zusammen mit dem umhüllenden, den oder die elektrischen Leiter nach außen und ggf. gegeneinander elektrisch isolierenden Isolationsschlauch sowie allen weiteren funktionellen Elementen, die mit der Leitung fest verbunden sind, verstanden. Die Elektrodenleitung umfasst in der Regel an ihrem distalen Ende auch eine sogenannte Elektrodenspitze, mittels der die elektrische Energie von dem oder den Leiter(n) in das zu behandelnde Gewebe eingeleitet wird. Häufig ist eine Elektrodenspitze mit Ankerelementen oder Haltestrukturen versehen, mit denen die Konstanz der räumlichen Lage der Übergangsstelle der elektrischen Energie in das zu behandelnde Gewebe sichergestellt wird. Elektrodenleitungen können auch ein oder mehrere weitere Elemente wie z. B. eine Ringelektrode oder eine Wendel umfassen, mittels derer elektrische Energie in das zu behandelnde Gewebe eingeleitet wird. Die Elektrodenspitze kann als Ableit-, Stimulations- oder Messelektrode ausgebildet sein. Weiter besitzt die Elektrodenleitung in der Regel, beispielsweise an ihrem proximalen Ende, einen Stecker, mit der die Elektrodenleitung mit einem Implantat verbunden werden kann, wobei der Stecker hierfür in eine entsprechende Buchse des Implantats eingesteckt wird. Der Stecker besitzt einen oder mehrere Anschlüsse, wobei jeder Anschluss mit einem elektrischen Leiter der Elektrodenleitung verbunden ist, Wobei dieser Leiter auch aus mehreren parallel geführten, gegeneinander isulierten Leitern bestehen kann. Entsprechend ist in der Buchse für jeden Anschluss der Elektrodenleitung ein Anschluss der Buchse vorgesehen.

An moderne Implantate, beispielsweise einen Mehrkammer-Herzschrittmacher, werden häufig mehrere Elektrodenleitungen angeschlossen. Hierbei besteht das Bestreben, die Elektrodenleitungen und ihre Anschlüsse möglichst dünn bzw. klein zu gestalten. Dies erschwert andererseits die gut sichtbare Markierung der Stecker und Anschlüsse sowie die Unterscheidung der Elektrodenleitungen. Darüber hinaus steigt mit zunehmender Anzahl von Elektrodenleitungen die Gefahr, dass einzelne Elektrodenleitungen verwechselt und/oder falsch angeschlossen werden. Deshalb ist es wünschenswert, wenn ein Implantat erkennt, welche Elektrodenleitungen angeschlossen sind, so dass es diese adäquat ansteuern kann. Zudem ist es zum Betreiben des Implantats hilfreich, wenn die Elektrodenleitungen und/oder ihre Eigenschaften für das Implantat identifizierbar sind.

Dokument US 2004/0073265 A1 beschreibt eine Vorrichtung, die eine Möglichkeit bietet, falsch verbundene Koronarleitungen und/oder falsche Verbindungen zu Herzrhythmusmanagement-Vorrichtungen zu erkennen. Dazu erzeugt eine Spannungseinbringungsvorrichtung eines Schrittmachers einen Spannungspuls zwischen einer Elektrode, die mit einer Leitung mit dem Schrittmacher verbunden ist, und einer Kopf- oder Gehäuseelektrode des Schrittmachers. Die Gehäuseelektrode sendet ein Verbindungssignal. Die Elektrode wird genutzt, um ein korrespondierendes Verbindungssignal unter Nutzung der Leitung zu messen. Ein Messmodul der Vorrichtung misst weiter eine oder mehrere Eigenschaften des korrespondierenden Verbindungssignals wie seine Stromstärke, Spannung, Impedanz und/oder sein Zeitverzug (nach Aussendung des Spannungspulses). Die Signaleigenschaften können von einer oder mehreren Leitungen und/oder vom übermittelnden Gewebe und Flüssigkeiten (beispielsweise einem Herz inklusive einer oder mehrere seiner Kammern), die dazwischen liegen, beeinflusst werden. Ein Vergleichsmodul des Schrittmachers kann daraufhin feststellen, ob die Leitung ordnungsgemäß zu einem Kontakt des Schrittmachers geführt ist, wobei eine oder mehrere Eigenschaften des korrespondierenden Verbindungssignals mit geeigneten vorgewählten Wertebereichen verglichen werden. Beispielsweise kann eine gemessene Impedanz mit einem erwarteten Impedanzbereich verglichen werden. Die in der Druckschrift beschriebene Vorrichtung identifiziert damit nicht selektiv die Leitung, sondern testet vielmehr, ob das korrespondierende Vergleichssignal, das über eine Leitung nach Anregung des Körpers durch einen Spannungspuls einer Gehäuseelektrode des Schrittmachers empfangen wird, Eigenschaften innerhalb eines vorgegebenen Wertebereichs aufweist. Die Eigenschaften des korrespondierenden Vergleichssignals werden auch durch das angeregte Körpergewebe zwischen der Gehäuseelektrode des Schrittmachers und der empfangenden Elektrode bestimmt. Nur sehr grobe Abweichungen, wie sie durch eine nicht verbundene oder vollkommen falsche Art von Leitung auftreten, können sicher auf die Leitung zurückgeführt werden, geringere Abweichungen können körperbedingt sein. Die oben genannte Vorrichtung kann damit die sichere und gezielte Erkennung und Unterscheidung von Elektrodenleitungen mit ähnlichen Eigenschaften nicht gewährleisten.

Eine ähnliche Vorrichtung wird auch in Dokument US 2006/0212083 A1 offenbart. Auch in dieser Druckschrift wird betont, dass die Signaleigenschaften von den Leitungen und/oder von dem/der dazwischen liegenden übermittelnden Gewebe und Flüssigkeit beeinflusst werden.

Dokument US 2011/0112609 A1 beschreibt ein System zur Rückenmarksstimulation mit mindestens einer implantierbaren Stimulationsleitung. Es umfasst insbesondere ein ärztliches Programmiergerät und einen implantierbaren Pulsgenerator, der mit einer oder mehreren implantierbaren Stimulationsleitungen verbunden ist, die je eine Vielzahl von Elektroden tragen. Die Stimulationsleitung weist ein oder zwei Leitungskörper auf. Die Elektroden passen genau in den Epiduralraum der Wirbelsäule. Da das dortige Gewebe leitend ist, können elektrische Messungen zwischen den Elektroden durchgeführt werden. Ein Kontrollschaltkreis des implantierbaren Pulsgenerators erfasst solche elektrische Messungen, so dass das ärztliche Programmiergerät automatisch die einzelnen Leitungskörper identifizieren kann, die mit dem implantierbaren Pulsgenerator verbunden sind. Die elektrischen Messungen des Kontrollschaltkreises zur Identifikation der verbundenen Leitungskörper sind Feldpotentiale. Der Kontrollschaltkreis kann auch die Impedanz an jeder Elektrode messen, um die Kopplungseffizienz zwischen der jeweiligen Elektrode und dem Gewebe zu bestimmen und um die Fehlerkennung bezüglich der Verbindung zwischen der Elektrode und dem analogen Ausgabeschaltkreis des implantierbaren Pulsgenerators zu bestimmen. Bei dem bekannten System ist von Nachteil, dass die Identifikation nicht durch den implantierbaren Pulsgenerator selbst, sondern durch ein zusätzliches ärztliches Programmiergerät erfolgt.

Dokument US 2012/0123496 A1 beleuchtet die Erkennung der Verbindung und die Identifikation des Typs einer implantierten Leitung für eine implantierte medizinische Vorrichtung. Die Vorrichtung weist einen Prozessor auf, der die Verbindung sowie den Leitungstyp der Leitung bestimmen kann. Zunächst prüft ein Signalmessmodul die Verbindung der Leitungen, indem es Werte elektrischer Parameter während eines Signals zwischen zumindest zwei Elektroden prüft, insbesondere der Impedanz. Eine oder mehrere Leitungen können darin integrierte, aktive Elektronik aufweisen, die einen oder mehrere darin integrierte modulare Schaltkreise beinhaltet, je nachdem, ob die Leitung unipolar oder multipolar ist. Jeder der modularen Schaltkreise ist in der Lage, eine Vielzahl von Elektroden der Leitung zu steuern, und schließt eine Schaltungsanordnung ein, die elektrisch mit einer oder mehreren Elektroden der Leitung verbunden ist. Als solcher wirkt jeder der modularen Schaltkreise einer Leitung als Schnittstelle zwischen der implantierten medizinischen Vorrichtung und den Elektroden, mit denen der modulare Schaltkreis verbunden ist. Für die Messung der Impedanz steuert der Prozessor der Vorrichtung den modularen Schaltkreis, sodass dieser einen Spannungspuls zwischen einer ersten und einer zweiten Elektrode liefert. Das Signalmessmodul misst den resultierenden Strom und der Prozessor leitet den Impedanzwert ab. In einem weiteren Schritt sendet der Prozessor ein Abfragesignal entlang eines ersten Leiters der Leitung, um eine Antwort von den modularen Schaltkreisen über eine zweite Leitung zu erhalten. Eine solche Antwort von jedem modularen Schaltkreis liefert dem Prozessor Informationen zu dem modularen Schaltkreis und den Elektroden, die er steuert. In einem weiteren Konfigurationsschritt sendet der Prozessor ein Signal über die erste Leitung. Der Konfigurationsschritt schließt ein, dass die aktive Konfiguration der modularen Schaltkreise programmiert wird. Bezüglich Leitungsausführungen und darin verwendeter aktiver Elektronik bzw. modularer Schaltkreise wird auf das Dokument US 7,713,194 verwiesen. Gemäß dieser Druckschrift ist der modulare Schaltkreis derart ausgestaltet, dass er durch einen Bus gesteuert wird. Die Druckschrift US 2012/0123496 A1 beschreibt demnach, dass die zusätzliche Schnittstellenelektronik modularer Schaltkreise erkannt und somit die Elektrodenleitung bestimmt werden kann. Bei der bekannten Vorrichtung ist nachteilig, dass komplexe modulare Schaltkreise mit aktiver Elektronik zur Steuerung der Elektroden implementiert und programmiert werden müssen. Ferner beziehen sich die Informationen nur auf die modularen Schaltkreise und die damit verbundenen Elektroden, nicht aber auf die Leitung als Ganzes.

Ein Verfahren und eine Vorrichtung zur automatischen Erkennung von implantierbaren medizinischen Leitungen und deren Konfiguration sind in dem Dokument US 2003/0018369 A1 dargestellt. Dafür ist ein erster Kommunikationsschaltkreis, der Daten wie Modell- und Seriennummer, technische Information und Kalibrierungsdaten speichert, mit der Leitung verbunden oder in ihr integriert. Dieser weist einen Empfänger und einen Sender auf, um Datensignale von einer externen Quelle zu empfangen. So kann er bei der Herstellung mit Identifikationsdaten, Kalibrierungsdaten und anderen Daten programmiert werden. Der erste Kommunikationsschaltkreis ist als passiver Transponder ausgeführt und weist neben dem Receiver und Transmitter weiter einen Energiekoppler zur Energieversorgung und einen Steuerschaltkreis auf, welcher mit einem nicht-flüchtigen Speicher verbunden ist. Der Steuerschaltkreis liefert die in dem Speicher gespeicherten Informationen der Leitung zum Transmitter/Receiver des Transponders, der die Daten via RF oder anderer Kommunikation übermittelt. Während der Implantation der Leitung oder danach können die Informationen zu einem zweiten Kommunikationsschaltkreis außerhalb der Leitung transferiert werden. Die transferierten Daten können zur Identifikation der Leitung genutzt werden, in eine Patientenakte aufgenommen und in einen Zentralspeicher für die Nutzung durch Gesundheitsdienstleister transferiert werden. Anhand des Transponders kann die Leitung automatisch erkannt werden und die im Speicher abgelegten Daten können direkt transferiert und weitergegeben werden. Der Transponder benötigt neben einem Transmitter und Receiver allerdings eine separate Energieversorgung, eine Steuereinheit und einen programmierbaren, digitalen Speicher. Dadurch ist der Gesamtaufbau der Leitung vergleichsweise aufwendig und teuer.

Auch Dokument US 2014/0343633 A1 stellt eine elektrisch identifizierbare Elektrodenleitung mit einem Identifikationsmodul dar, welches zumindest einen Filter, einen Stromkonverter, eine Kommunikationsschaltung, einen Lastschalter und eine Speichereinheit wie ein EPROM zur Speicherung eines Identifikationscodes aufweist. Vor der Einbringung des Implantats wird jede Leitung implantiert und mit dem implantierbaren Pulsgenerator (oder einem externen Pulsgenerator) verbunden, welcher dann selbstidentifizierende Daten vom Identifikationsmodul ausliest und diese Information an eine externe Vorrichtung wie den Arztprogrammierer übermitteln kann. Dafür kann das Identifikationsmodul bis zu 32 Byte Daten speichern. Dieses Verfahren wird für jede Leitung, die implantiert wird, wiederholt. Das Identifikationsmodul nutzt zwei vorhandene Kontakte der Leitung zur Verbindung mit dem implantierbaren Pulsgenerator. Wie im zuvor genannten Dokument wird auch für diese bekannte Elektrodenleitung ein digitaler Speicher benötigt und der Aufbau des Identifikationsmoduls ist ähnlich komplex.

Aus den Dokumenten US 2006/0212096 A1, US 2008/0065181 A1 und US 7,983,763 B2 sind Vorrichtungen für die Identifizierung einer implantierbaren medizinischen Einrichtung und eines implantierten Leitersystems bekannt, bei dem ein RFID-Tag mit einem RFID-Chip in der Isolation, welche den Leiter umgibt, oder in dem Header einer implantierbaren Vorrichtung angeordnet ist. Ferner ist eine Leseeinrichtung vorgesehen, welche die in dem RFID-Chip gespeicherten Daten über das Gerät, das Leitersystem, den Hersteller oder den Patienten kabellos auslesen kann. Die auslesbaren Informationen enthalten jedoch keine Angaben über die aktuelle Anordnung und/oder den Anschluss der jeweiligen Elektroden. Zudem ist es bei den in diesen Dokumenten erläuterten Lösungen von Nachteil, dass vergleichsweise viel Energie für das Abfragen der Daten aus dem Implantat und für die Aktivierung des Chips aufgewendet werden muss. Hierfür werden zusätzliche Geräte eingesetzt, die eine erhebliche SAR (specific absorption rate - Maß für die Absorption eines elektromagnetischen Feldes durch das Gewebe)-Belastung des Patienten darstellen.

Dokument US 2007/203547 A1 zeigt Vorrichtungen und Methoden um auf in einem Implantat gespeicherte Information zuzugreifen.

Die Aufgabe der vorliegenden Erfindung besteht deshalb darin, ein einfaches Verfahren zur Identifikation einer Elektrodenleitung anzugeben, welches eine eindeutige Zuordnung einer Elektrodenleitung zu einem Anschluss des Implantats ermöglicht. Die Aufgabe besteht ferner darin, ein entsprechendes Implantat mit einer Elektrodenleitung zu schaffen.

Die für die ausführung der erfindung nutzbare Elektrodenleitung besitzt ein durch den Isolationsschlauch oder den Stecker hermetisch abgeschlossenes passives RFID-Inlay mit einem RFID-Chip und einer mit dem RFID-Chip elektrisch leitend verbundenen Antenne, wobei die Antenne elektromagnetisch mit dem mindestens einen elektrischen Leiter gekoppelt ist. Vorzugsweise erfolgt die Kopplung derart, dass innerhalb des RFID-Chips die nötige Betriebsleistung zum Auslesen der gespeicherten Informationen erreicht wird.

Die nötige Betriebsleistung zum Auslesen der innerhalb des RFID-Chips gespeicherten Informationen hängt vom individuell eingesetzten Chip ab. Nach heutigem Stand der Technik sind Chips verfügbar, bei denen eine Betriebsleistung von 0,008 mW zum Auslesen der innerhalb des RFID-Chips gespeicherten Informationen ausreicht. Durch zukünftig verfügbare RFID-Chips kann diese Schwelle für die zum Auslesen der gespeicherten Informationen notwendige Leistung weiter sinken.

Durch diese Elektrodenleitung können auf einfache und kostengünstige Weise Informationen die Elektrodenleitung betreffend einzeln ausgelesen werden, ohne einen therapeutischen Pfad der Elektrodenleitung durch eine galvanische Kopplung zu gefährden. Zudem ist keine zusätzliche Durchführung am aktiven Implantat für eine Sendeantenne notwendig und der Leistungsbedarf zum Auslesen der Informationen ist gegenüber einem Auslesen von Informationen über eine am aktiven Implantat befestigte Sendeantenne deutlich reduziert. Hierdurch wird auch die SAR-Belastung des Patienten verringert.

Das Auslesen des RFID-Chips des RFID-Inlays erfolgt vom aktiven Implantat mittels der einen oder mehreren von elektrischen Signalen, der Stimulation und/oder der Messung von elektrischen Signalen des Körpers, in den die Elektrodenleitung implantiert ist, und die (sekundäre) Funktion als Antenne.

Die Lösung hat den Vorteil, dass die Elektrodenleitung ihren therapeutischen und mechanischen Anforderungen weiterhin uneingeschränkt genügt. Hierbei ist es besonders zweckmäßig, dass die Materialien für die elektrischen Leitung(en) nicht verändert werden müssen, da diese Materialien die mechanische Robustheit einer Elektrodenleitung gewährleisten. Zudem bleibt auch die für den therapeutischen Einsatz der Elektrodenleitung wichtige Flexibilität der Elektrodenleitung erhalten.

Die Erfinder haben erkannt, dass, um die Identifizierung des Anschlusses und der Anordnung einer Elektrodenleitung zu ermöglichen, eine Änderung der technischen Eigenschaften einer Elektrodenleitung problematisch ist, ohne die Funktion und Haltbarkeit der Elektrodenleitung hinsichtlich ihrer therapeutischen und mechanischen Anforderungen zu beeinträchtigen. Hierfür wurde eine Informationsübertragung mittels RFID als prinzipiell geeignet angesehen. Die Erfinder haben in diesem Zusammenhang jedoch festgestellt, dass die Materialien für die elektrischen Leitung(en) einen hohen spezifischen Widerstand aufweisen. Für die notwendige Flexibilität der Elektrodenleitung reduziert man deren Isolationsstärke auf ein nötiges Minimum. Die Erfinder kamen zu dem Ergebnis, dass diese Gestaltung einer Elektrodenleitung sowie der Betrieb im Körpergewebe, was ein stark verlustbehaftetes Medium darstellt, dazu führen, dass Elektrodenleitungen schlechte Antenneneigenschaften aufweisen, die für die Übertragung von Informationen auf so geringem Leistungsniveau, wie für RFID notwendig, als unzureichend eingestuft werden.

Die obige Analyse brachte die Erfinder dazu, sich mit der elektromagnetischen Kopplung eines RFID-Elements mit dem mindestens einen elektrischen Leiter der Elektrodenleitung zu beschäftigen, um die gewünschte Identifizierung des Anschlusses und der Anordnung einer Elektrodenleitung mittels dieser Technologie zu realisieren.

Trotz der unzureichend erscheinenden Antenneneigenschaften von Elektrodenleitungen für die Nachrichtenübertragung mittels RFID-Technologie, führt eine spezielle Abstimmung der Gestaltung der Antenne eines RFID-Inlays auf die jeweils vorgegebene Gestalt der Elektrodenleitung gemeinsam mit der Anordnung der Antenne des RFID-Inlays in unmittelbarer Nachbarschaft zu dem elektrischen Leiter der Elektrodenleitung zu ausreichender elektromagnetischer Kopplung, so dass am RFID-Inlay auch bei geringem Energieaufwand des aktiven Implantats ausreichend Signalstärke zum Auslesen der hiermit übertragenen Informationen verbleibt.

Hierdurch wurde eine Nahfeldkopplung realisiert, die wenig vom umliegenden verlustbehafteten Medium (Körpergewebe) beeinflusst ist. Die Erfinder haben erkannt, dass für unterschiedliche Elektrodenleitungstypen die optimale Antennenform für das RFID-Inlay individuell angepasst werden muss. Zusätzlich ist die optimale Antennenform abhängig von der Art der dominanten Kopplung. Eine vorwiegend magnetische Kopplung erfordert ein anderes Design, als eine vorrangig elektrische oder elektromagnetische Kopplung.

In einer besonders bevorzugten Ausführung entspricht, insbesondere für eine elektrische Kopplung zwischen Antenne und Leiter, die Form der Antenne des RFID-Inlays der Form des mindestens einen elektrischen Leiters der Elektrodenleitung. Die Erfinder haben diesbezüglich festgestellt, dass bei einem geraden seilförmigen elektrischen Leiter, wie bei Tachykardie-Elektrodenleitungen, im Falle einer elektrischen Kopplung ein Dipoldesign zu bevorzugen ist. Für eine magnetische Kopplung ist dagegen eine Gestaltung mit einer höheren Induktivität, beispielsweise in Ring- oder Spiralform, vorteilhaft. Bei spiralförmigen elektrischen Leitern bieten sich für eine vorrangig elektrische Kopplung Antennenformen an, die ebenfalls eine wendelförmige Struktur aufweisen.

In einer besonders bevorzugten Ausführungsform entspricht die Steigung der Wendel der Antenne des RFID-Inlays der Steigung der Wendel des mindestens einen elektrischen Leiters der Elektrodenleitung. Eine spezielle Situation ergibt sich diesbezüglich bei Elektrodenleitungen mit zwei spiralförmigen Leitern, wobei beide Wendel eine unterschiedliche Steigung aufweisen. Für eine optimale Kopplung an beide Leiter ist es vorteilhaft, wenn die Steigung der Wendel der Antenne des RFID-Inlays dem Mittelwert der Steigungen der beiden Wendel der elektrischen Leiter der Elektrodenleitung entspricht.

Die Lösung hat weiter den Vorteil, dass die Dämpfungsverluste gering sind. Die implantierten Elektrodenleitungen können insbesondere gezielt einzeln ausgelesen werden, was eine Zuordnung der Informationen der elektrischen Elektrodenleitung zur Lage dieser Elektrodenleitung (zum Beispiel atrial, ventrikulär) ermöglicht. Eine galvanische Kopplung von RFID-Inlay, insbesondere der Antenne des RFID-Inlays, und einer elektrischen Leitung der Elektrodenleitung besteht nicht.

Die Energie, die mittels Antenne in den elektrischen Leiter beziehungsweise durch den elektrischen Leiter in die Antenne des RFID-Inlays drahtlos eingekoppelt wird, ist hierbei in vorteilhafter Weise so bemessen, dass bei einer Übertragung dieser Energie auf eine Stelle im Gewebe des Patienten die dadurch auftretenden Temperaturen einen Maximalwert von 47 °C nicht überschreiten (Grenzwerte für elektromagnetische Strahlung, die in den menschlichen Körper abgegeben werden darf, finden sich z. B. in: DIN EN 62209-1:2007-03 oder VDE 0848-209-1:2007-03). Zudem sind die verwendeten Amplituden stets kleiner als die im jeweiligen, für die Informationsübertragung genutzten Frequenzbereich (zum Beispiel 840 MHz bis 960 MHz) geltenden Grenzwerte für ungewollte Stimulation. Zur Bewertung der elektromagnetischen Verträglichkeit von Implantaten zur Behandlung von Tachikardien, Bradykardien oder von Geräten für die kardiale Resynchronisationstherapie sei auf ANSI/AAMI/ISO 14117/Ed.l verwiesen.

Das RFID-Inlay kann entweder in den Stecker der Elektrodenleitung eingebettet und/oder an oder in dem Isolationsschlauch angeordnet sein. Wenn das RFID-Inlay an der Außenseite des Isolationsschlauchs angebracht ist, wird vorzugsweise eine Isolationshülse verwendet, welche den Isolationsschlauch umgibt und das RFID-Inlay hermetisch abschließt. Hierfür ist das RFID-Inlay zwischen Isolationsschlauch und Isolationshülse und/oder in der Isolationshülse angeordnet. In dem Fall, dass das RFID-Inlay in dem Isolationsschlauch vorgesehen ist, so ist das RFID-Inlay hermetisch abgeschlossen in dem Isolationsschlauch eingebettet.

In einer bevorzugten Ausführung weist der RFID-Chip eine vorzugsweise mehrfach beschreibbare Speichereinheit zum Speichern von über die Antenne übertragenen Informationen auf, zum Beispiel 512 Bit frei beschreibbar und 240 Bit für den Electronic Product Code (EPC). Zusätzlich können Informationen in der Speichereinheit gespeichert werden, die werksseitig vorgegeben werden. Auch diese Informationen können über die Antenne des RFID-Inlays und den elektrischen Leiter der Elektrodenleitung an das Implantat übertragen werden. Die in dem Speicher gespeicherten Informationen können beispielsweise umfassen: Hersteller, Typ der Elektrodenleitung, Seriennummer, Herstellungsdatum, Zulassungsregion, Zulassungsbedingungen, Implantationsdatum, Implantationskompatibilität, MRI-Kompatibilität und dergl. Weiter kann der Speicher Sicherheitsmechanismen und Sicherheitsinformationen enthalten, die die Integrität (zum Beispiel bei partiellem Datenverlust) und/oder die Echtheit (beispielsweise bei Manipulation) der gespeicherten Informationen sicherstellen beziehungsweise erkennbar machen.

Es ist weiter von Vorteil, dass die Antenne des RFID-Inlays als Drahtschleife, als offener Ring, als Spiralabschnitt, als durchtrennte (geschlitzte) Metallhülse oder dergl. ausgebildet ist. Eine derartige Antenne ist einfach und kostengünstig realisierbar.

In einer weiteren Ausführung ist der mindestens eine elektrische Leiter und/oder die Anordnung der Antenne des RFID-Inlays derart eingerichtet beziehungsweise konzipiert, dass an der Stelle der elektromagnetischen Kopplung des elektrischen Leiters mit der Antenne des RFID-Inlays in einem vorgegebenen Frequenzbereich (zum Beispiel 840 bis 960 MHz) ein Strom- oder Spannungsbauch der Schwingung erzeugbar ist. Dies ermöglicht eine Verbesserung der magnetischen beziehungsweise elektrischen Kopplung zwischen der Antenne des RFID-Inlays und des als Antenne wirkenden elektrischen Leiters. Hierfür kann die Elektrodenleitung entweder hinsichtlich ihrer Abmessungen und/oder elektrischen Parameter schwingungsgünstig gestaltet und/oder die elektrische Leitung implantatsseitig so abgeschlossen werden, das heißt mit einem entsprechenden Impedanzwert versehen werden, dass sich das gewünschte Schwingungsverhalten in dem vorgegebenen Frequenzbereich ergibt.

Die Erfinder haben weiter erkannt, dass aufgrund der hohen Dämpfung elektromagnetischer Wellen in biologischem Gewebe die Ausbildung stehender Wellen auf der Elektrodenleitung zwar weniger ausgeprägt ist, bei der Platzierung des RFID-Inlays jedoch darauf zu achten ist, dass sich dieses nicht am Ort eines potentiellen Wellenknotens befindet. Die Position des Knotens ist dabei abhängig vom Design der Elektrodenleitung (z.B. Wickeldichte) bzw. der sich daraus ergebenden Wellenimpedanz und der entsprechenden Eingangsimpedanz am aktiven Implantat. Die optimale Platzierung ist deswegen für jeden Elektrodentyp gesondert festzustellen. Sie ist vorzugsweise so vorzunehmen, dass das RFID-Inlay, insbesondere dessen Antenne, in der Mitte zwischen zwei Wellenknoten angeordnet ist. Generell ist wegen der Dämpfung eine Platzierung des RFID-Inlays möglichst in unmittelbarer Nähe zum aktiven Implantat vorteilhaft.

Die obige Aufgabe wird ferner durch ein Verfahren zur Identifikation einer oben beschriebenen Elektrodenleitung mittels eines Implantats gemäß Anspruch 1 gelöst.

Das erfindungsgemäße Verfahren erlaubt auf einfache und kostengünstige Weise eine Erkennung des oder der elektrischen Leiter einer Elektrodenleitung bzw. das Auslesen von Informationen über die Elektrodenleitung. Beispiele derartiger Informationen sind oben angegeben.

In einem bevorzugten Ausführungsbeispiel kann die Erzeugung des Abfragesignals durch die Steuereinrichtung in regelmäßigen und/oder vorgebbaren Zeitabständen und/oder nach dem Auftreten eines vorbestimmten Ereignisses erfolgen. Derartige Ereignisse können z.B. sein: der Austausch einer Elektrodenleitung, eine jährliche/monatliche Inventarisierung der Implantate über eine Telemonitoring-Plattform, eine definierte Änderung einer elektrischen Eigenschaft einer Elektrodenleitung (z. B. infolge eines Elektrodendefekts).

Häufig verfügt ein Implantat über besonders für hohe Frequenzen filternd wirkende EMI-Kondensatoren (Kondensatoren zur Funkentstörung - electromagnetic interference). Diese wirken beim erfindungsgemäßen Verfahren störend, sodass es notwendig ist, wenn während des Sendens des Abfragesignals und des Empfangens des Antwortsignals zur effizienteren Energieeinbringung und Signalausbeute die filternde Wirkung von EMI-Kondensatoren des Implantats deaktiviert wird. Beispiele zur Realisierung der Deaktivierung werden unten erläutert.

Die obige Aufgabe wird ferner gelöst durch ein Implantat mit einer Elektrodenleitung gemäß Anspruch 3.

Ein derartiges aktives Implantat ermöglicht die einfache und kostengünstige Feststellung des Zustandes der angeschlossenen Elektrodenleitungen.

Um die elektrischen Leiter der Elektrodenleitung einzeln ansprechen zu können, ist es von Vorteil, dass, wenn die Buchse eine Vielzahl von Anschlüssen zur Verbindung mit den elektrischen Leitern einer oder mehrerer Elektrodenleitungen aufweist, eine Sende- und/oder Empfangseinheit der Steuereinrichtung vorgesehen ist, welche über einen Multiplexer mit der Vielzahl von Anschlüssen verbunden ist.

Wie oben beschrieben wurde, ist esnotwendig, die filternde Wirkung von EMI-Kondensatoren zu unterbinden. Dies erfolgt erfindungsgemäß dadurch, dass ein Schalter vorgesehen ist, welcher eine Verbindung zwischen einem EMI-Kondensator und dem mit der Steuereinrichtung verbundenen elektrischen Leiter öffnet. Alternativ ist ein Schalter vorgesehen, welcher eine Parallelschaltung von einer oder mehrerer Spulen mit einem EMI-Kondensator bewirkt, sodass ein Parallelschwingkreis ausgebildet wird, wobei die Spulen derart dimensioniert sind, dass der Parallelschwingkreis eine Resonanz bei einer der Arbeitsfrequenzen der Steuereinrichtung aufweist.

Wie oben bereits dargestellt wurde ist es von Vorteil, dass die Steuereinrichtung derart eingerichtet ist, dass sie das Abfragesignal in regelmäßigen und/oder vorgebbaren Zeitabständen und/oder nach dem Auftreten eines vorbestimmten Ereignisses erzeugt.

In einem Ausführungsbeispiel ist die Steuereinrichtung derart eingerichtet, dass sie das Abfragesignal in regelmäßigen und/oder vorgebbaren Zeitabständen und/oder nach dem Auftreten eines vorbestimmten Ereignisses erzeugt

Die Erfindung wird nachfolgend anhand von Ausführungsbeispielen und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: ein Ausführungsbeispiel einer erfindungsgemäßen Elektrodenleitung, die mit einem erfindungsgemäßen Implantat verbunden ist, in einer teilweise geschnittenen perspektivischen Ansicht von der Seite,
- Fig. 2: ein RFID-Inlay der Elektrodenleitung gemäß Fig. 1,
- Fig. 3: ein zweites Ausführungsbeispiel einer erfindungsgemäßen Elektrodenleitung in einer perspektivischen Ansicht von der Seite,
- Fig. 4A-7B: weitere Ausführungsbeispiele von RFID-Inlays,
- Fig. 8: eine Schaltskizze des Implantats gemäß Fig. 1,
- Fig. 9: eine Schaltskizze eines weiteren Ausführungsbeispiel eines erfindungsgemäßen Implantats,
- Fig. 10A-B: zwei Beispiele für die Anordnung von RFID-Inlays neben einem oder mehreren elektrischen Leitern einer erfindungsgemäßen Elektrodenleitung und
- Fig. 11: die Aktivierung des RFID-Inlays durch ein externes Gerät.

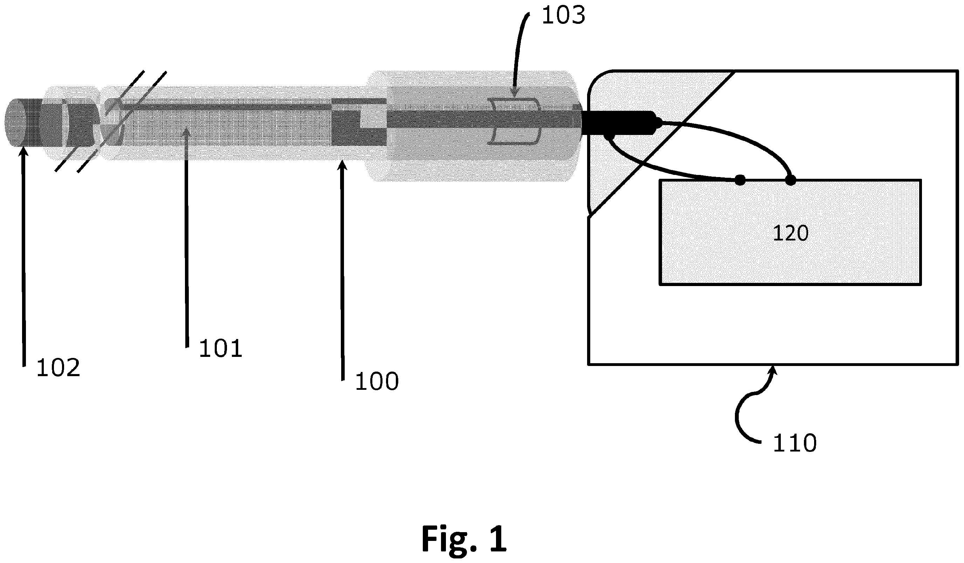

Figur 1 zeigt schematisch ein Ausführungsbeispiel einer Elektrodenleitung 100 mit einem einzigen elektrischen Leiter 101, welcher von einem elektrisch isolierenden Isolationsschlauch 310 umhüllt ist. Am distalen Ende der Elektrodenleitung 100 ist eine Elektrodenspitze 102 angeordnet, welche den elektrischen Kontakt zur Umgebung, beispielsweise dem Gewebe eines Patienten, herstellt. Die Elektrodenspitze kann als Stimulations-, Mess- oder Ableitelektrode ausgebildet sein. Am proximalen Ende der Elektrodenleitung 100 ist ein Stecker 330 angeordnet, welcher in die Buchse eines aktiven Implantats 110 eingesteckt ist. Das aktive Implantat 110 kann beispielsweise als Herzschrittmacher oder Defibrillator ausgebildet sein. Nach der Herstellung einer mechanischen und elektrischen Verbindung zwischen Stecker 330 und Buchse des Implantats 110 besteht über entsprechende Anschlüsse des Steckers 330 beziehungsweise der Buchse eine elektrisch leitende Verbindung zwischen dem elektrischen Leiter 101 der Elektrodenleitung 100 und den innenliegenden elektrischen Komponenten des Implantats 110, beispielsweise einer Steuereinrichtung 120.

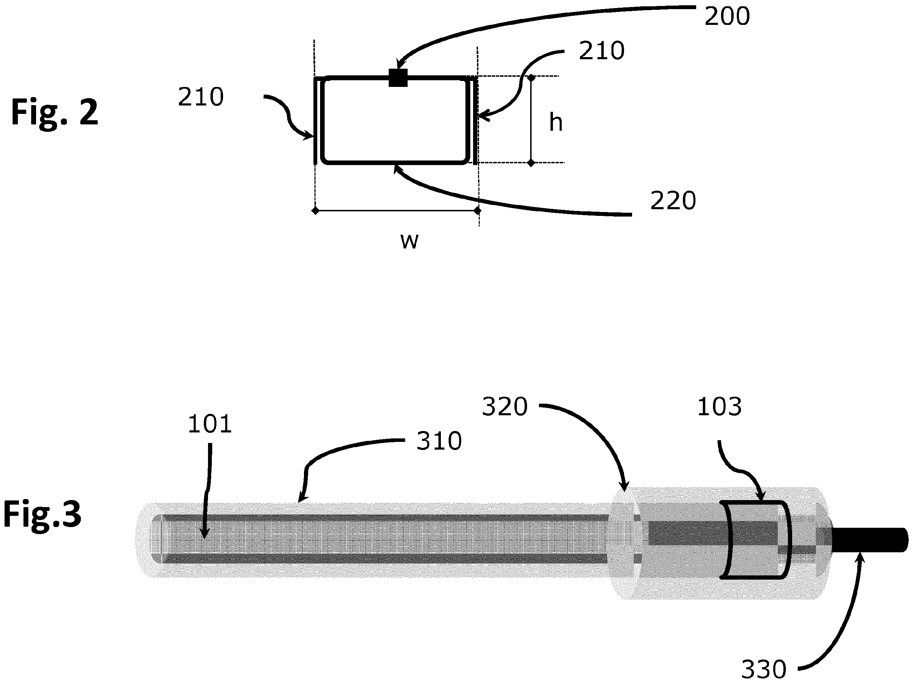

Wie in Figur 3 zu sehen ist zwischen dem beispielsweise als Silikonschlauch ausgeführten Isolationsschlauch 310 und einer am proximalen Ende der Elektrodenleitung 100 in der Nähe des Steckers 330 vorgesehenen Isolationshülse 320 ein passives RFID-Inlay 103 angeordnet. Hierbei umgibt die Isolationshülse 320, welche beispielsweise aus einem Flüssigkristallpolymer (LCP), Silikon, einer Keramik und/oder Glas besteht, derart den Isolationsschlauch 310, dass das RFID-Inlay 103 hermetisch gegenüber der Umgebung abgeschlossen ist.

Das RFID-Inlay kann auch in den Stecker 330 integriert sein. Dies hat den Vorteil, dass das Inlay nach dem Einstecken des Steckers 330 in die Buchse des Implantats innerhalb eines steifen Headers des Implantats 110 angeordnet ist, in dem die Buchse vorgesehen ist, und somit vor hoher mechanischer Belastung (Biegung/Abrieb) geschützt ist. Ein weiterer Vorteil besteht darin, dass eine räumlich nähere Anordnung des RFID-Inlays in Bezug auf das Implantat 110 geringere Verluste beim Auslesen und damit eine geringere RF-Belastung für den Patienten bedeuten.

Ein Ausführungsbeispiel für ein RFID-Inlay 103 ist in Figur 2 dargestellt. Es ist beispielsweise als ca. 1 cm² großes passives RFID-Inlay ausgeführt (Abmessungen zum Beispiel Breite w = 16 mm, Länge h = 6 mm, Dicke d < 0,1 mm) und weist einen RFID-Chip 200 sowie eine den RFID-Chip 200 schlaufenförmig umgebende Induktivität 220 zur Impedanzanpassung auf. Ferner ist eine Antenne 210 vorgesehen, welche in diesem Ausführungsbeispiel zwei Segmente besitzt. Die Antenne 210 kann beispielsweise mittels einer Metallisierung einer Kunststofffolie, vorzugsweise mit Gold oder Platin, hergestellt sein, wobei die Metallisierung auch auf der Innenseite der Isolationshülse 320, beispielsweise einer Keramik- oder Glashülse, angebracht sein kann.

Als RFID-Chip 200 kann zum Beispiel ein Chip für den Frequenzbereich zwischen 840 MHz und 960 MHz verwendet werden. Weiter kann eine Speicherkapazität von 512 Bit frei beschreibbar und 240 Bit zur Hinterlegung des Electronic Product Code (EPC) vorgesehen sein. Der Speicher des RFID-Chips 200 kann sowohl bei der Herstellung im Werk als auch während der Ausführung des erfindungsgemäßen Verfahrens zur Identifikation einer Elektrodenleitung gelesen und/oder beschrieben werden. Im Speicher des RFID-Chips 200 können Informationen zur Identifikation der Elektrodenleitung 100 sowie zu Ihrer Verwendung enthalten sein. Derartige Informationen können umfassen: den Hersteller, den Typ der Elektrodenleitung, die Seriennummer, das Herstellungsdatum, Zulassungsregionen, Zulassungsbedingungen, Implantationsdatum, Implantationskompatibilitäten, MRI-Kompatibilität und dergleichen. Weiter kann der Speicher des RFID-Chips 200 Sicherheitsmechanismen und Sicherheitsinformationen enthalten, die die Integrität (zum Beispiel bei partiellem Datenverlust) und die Echtheit (zum Beispiel bei Manipulation) der gespeicherten Informationen sicherstellt beziehungsweise erkennbar macht.

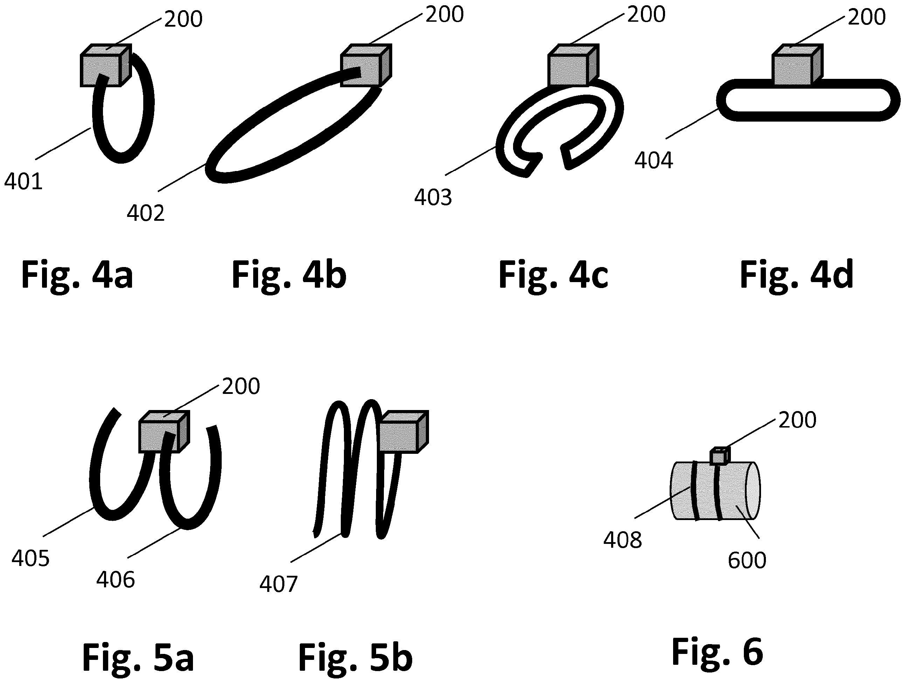

Die Figuren 4 bis 7 zeigen Ausführungsbeispiele für induktive und kapazitive Elemente der Antenne.

In Figur 4A ist eine ringförmige Antenne 401 dargestellt, welche zum Beispiel parallel zur Umfangsrichtung der Elektrodenleitung 100 verläuft. Dem gegenüber ist das in Figur 4B gezeigte Ausführungsbeispiel der Antenne 402 als Drahtschlaufe ausgebildet, welche schräg zur Umfangsrichtung der Elektrodenleitung 100 angeordnet ist. Bei dem in Figur 4C dargestellten Ausführungsbeispiel beschreibt die Antenne die Form eines offenen Rings, verläuft hierbei entlang eines geschlossenen Weges, zunächst auf der Außenseite des offenen Rings und anschließend an der Innenseite dieses Rings. Figur 4D zeigt eine ovale Drahtschlaufe als Antenne 404, welche parallel zur Längsrichtung (und damit senkrecht zur Umfangsrichtung der Elektrodenleitung 200 verläuft.

Die in den Figuren 5A und 5B dargestellten Antennen 405, 406 und 407 sind als Spiralabschnitte eines Drahtes gestaltet. Hierbei weist das in Figur 5A dargestellte Ausführungsbeispiel zwei separate, gegenüberliegende Drahtspiralabschnitte 405, 406 auf, während das in Figur 5B gezeigte Ausführungsbeispiel lediglich einen einzigen Drahtspiralabschnitt 407 besitzt.

In Figur 6 ist gezeigt, dass ein Spiralabschnitt einer Antenne 408, die analog zu Fig. 5B aufgebaut ist, auch als Metallisierung auf einer Keramik- oder Glashülse 600 aufgebracht sein kann.

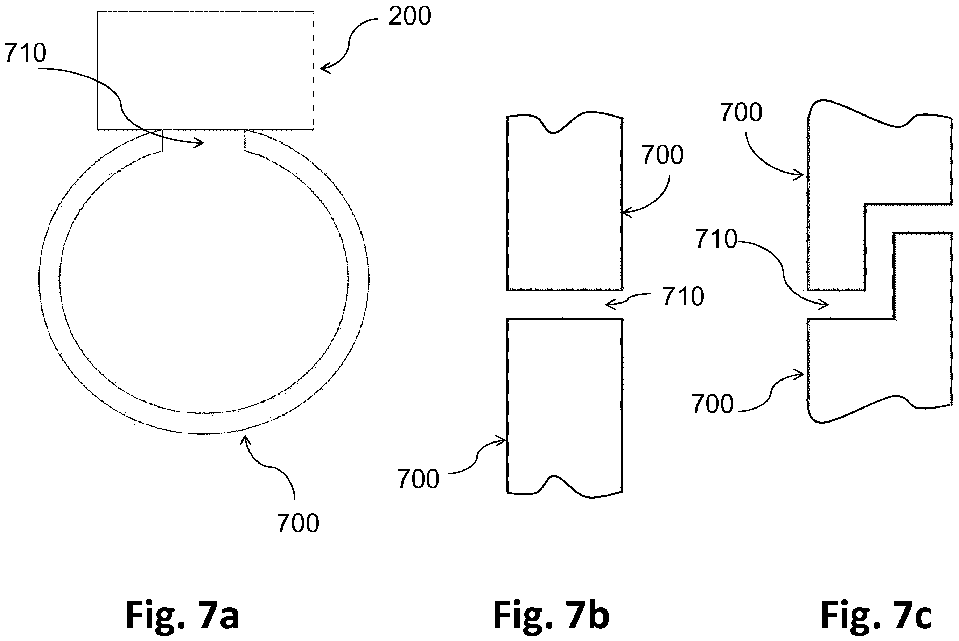

In der Figur 7 ist die Antenne des RFID-Inlays als geschlitzte Metallhülse 700 ausgeführt. Hierbei ist der Schlitz mit dem Bezugszeichen 710 versehen. Die Figuren 7B und 7C zeigen zwei Ausführungsbeispiele für den Schlitz 710. Das in Figur 7B dargestellte Ausführungsbeispiel zeigt lediglich einen geradlinigen, quer zur Umfangsrichtung der Metallhülse 700 verlaufenden Schlitz 710, während das in Figur 7C gezeigte Ausführungsbeispiel einen Schlitz 710 aufweist, der aus drei Abschnitten besteht, wobei ein erster und ein dritter Abschnitt quer zur Umfangsrichtung der Metallhülse 700 verlaufen und ein zweiter, mittlerer Abschnitt in Umfangsrichtung.

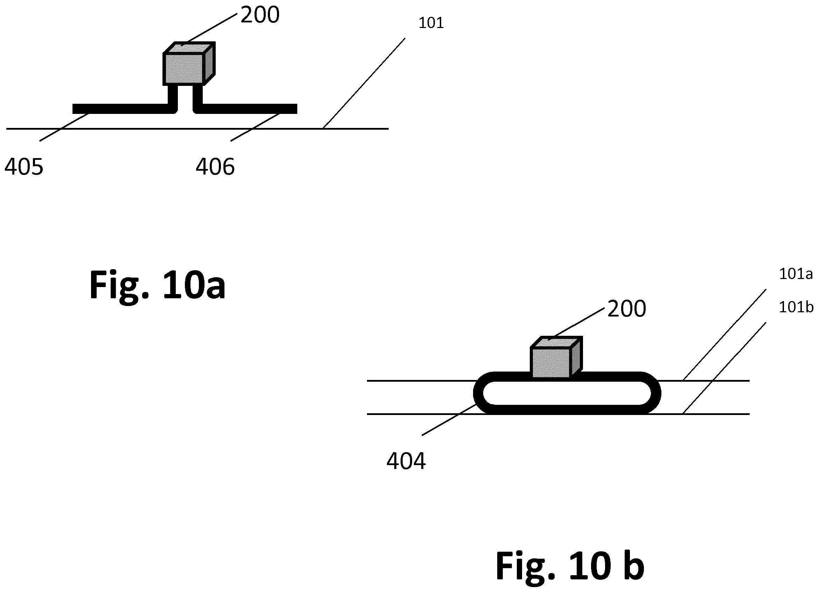

Es ist vorgesehen, dass die Antenne des RFID-Inlays 103 in der Elektrodenleitung 100, die mit dem RFID-Chip 200 verbunden ist, elektromagnetisch mit dem elektrischen Leiter 101 gekoppelt ist. Eine vorteilhafte Anordnung von Antenne 405, 406 und elektrischem Leiter 101 hierfür ist in einem Ausführungsbeispiel in Figur 10A dargestellt. Die Antenne 405, 406 ist hier als Dipol ausgeführt, sodass eine bevorzugt elektrische Kopplung zwischen der Antenne 405, 406 des RFID-Inlays 103 mit dem elektrischen Leiter 101 der Elektrodenleitung 100 erfolgt. Alternativ kann, wie in Figur 10B dargestellt, eine bevorzugte magnetische Kopplung zwischen der Antenne 404 des RFID-Inlays 103 (siehe auch Figur 4D) und einem ersten elektrischen Leiter 101a und einem zweiten elektrischen Leiter 101b der Elektrodenleitung 100 realisiert werden.

Das Implantat 110 enthält, wie oben bereits erwähnt, eine hermetisch nach außen abgeschlossene Steuereinrichtung 120, welche mit dem Anschluss oder den Anschlüssen für die Elektrodenleitung 100 verbunden ist. Nach Einstecken einer Elektrodenleitung 100 in eine entsprechende Buchse besteht daher auch eine elektrische Verbindung zwischen der Steuereinrichtung 120 und dem einen elektrischen Leiter 101 oder den mehreren elektrischen Leitern 101 der Elektrodenleitung 100. Um die Elektrodenleitung 100 abzufragen, gibt die Steuereinrichtung 120 ein hochfrequentes Abfragesignal in einem geeigneten Frequenzbereich (zum Beispiel 860 MHz) auf den auszulesenden elektrischen Leiter 101 der Elektrodenleitung 100. Der elektrische Leiter 101 wird erfindungsgemäß als Sendeantenne verwendet, sendet das Abfragesignal, das in die Antenne 210 des RFID-Inlays 103 koppelt und hierdurch den RFID-Chip 200 aktiviert. Durch das Abfragesignal wird elektromagnetische Energie in den RFID-Chip 200 übertragen. Das Abfragesignal wird durch den RFID-Chip 200 in bekannter Weise verarbeitet und ein entsprechendes Antwortsignal generiert, welches durch die Antenne 210 des RFID-Inlays 103 gesendet wird. Das Antwortsignal koppelt in den als Antenne wirkenden elektrischen Leiter 101 der Elektrodenleitung 100, wird von dort an die Steuereinrichtung 120 des Implantats 110 weitergeleitet und dort verarbeitet. Das Antwortsignal wird beispielsweise für die richtige Ansteuerung des/der elektrischen Leiter(s) 101 der Elektrodenleitung 100 benötigt.

Durch die räumlich nahe Anordnung zwischen dem hermetisch gekapselten RFID-Inlay 103 und der elektrischen Leitung 101 (siehe Figuren 10A und 10B) ist auch bei schlechter Anpassung eine gute elektromagnetische Kopplung möglich.

Es wird in vorteilhafter Weise eine Nahfeldkopplung realisiert, die wenig vom umliegenden verlustbehafteten Medium (Körpergewebe) beeinflusst ist. Für unterschiedliche Elektrodenleitungstypen, je nachdem, ob der mindestens eine elektrische Leiter spiralförmig ist, wie bei Bradykardie-Elektrodenleitungen üblich, oder in Form eines geraden seilförmigen elektrischen Leiters vorliegt, wie bei Tachykardie-Elektroden, sind optimale Antennenformen vorzusehen. Wie bereits oben erläutert wurde, ist die optimale Antennenform zusätzlich abhängig von der Art der dominanten Kopplung. Eine hauptsächlich magnetische Kopplung erfordert ein anderes Design, als eine vorrangig elektrische Kopplung. Beispielsweise ist bei einem geraden seilförmigen elektrischen Leiter, wie bei einer Tachykardie-Elektrodenleitung, im Falle einer elektrischen Kopplung ein Dipoldesign, wie in Figur 10A dargestellt, zu bevorzugen. Für eine magnetische Kopplung ist dagegen Design 10B vorteilhaft. Bei spiralförmigen Leitern bieten sich für eine vorrangig elektrische Kopplung Antennenformen an, die ebenfalls eine wendelförmige Struktur aufweisen, wie in Abbildung 5A/B und 6 skizziert. Die Steigung der Wendel der Antenne des RFID-Inlays richtet sich nach der Steigung der Wendel der beiden elektrischen Leiter zur Elektrodenspitze und zur Ringelektrode. Für eine optimierte Kopplung an beide Leiter ist es vorteilhaft, wenn die Steigung der Wendel der RFID-Antenne dem Mittelwert der Steigung der Wendel der Leiter zur Elektrodenspitze und Ringelektrode entspricht. Eine optimierte magnetische Kopplung im Falle eines spiralförmigen elektrischen Leiters ließe sich z.B. durch ein Design gemäß Abbildung 4C erreichen.

Bei der Möglichkeit der Verbindung mehrerer Elektrodenleitungen 100 beziehungsweise elektrischer Leiter 101 mit dem Implantat 110 kann das Implantat 110 einen Multiplexer aufweisen, welcher die Steuereinrichtung 120 mit dem jeweiligen Anschluss oder den jeweiligen Anschlüssen der Buchse des Implantats 110 verbindet, der oder die ausgelesen werden sollen.

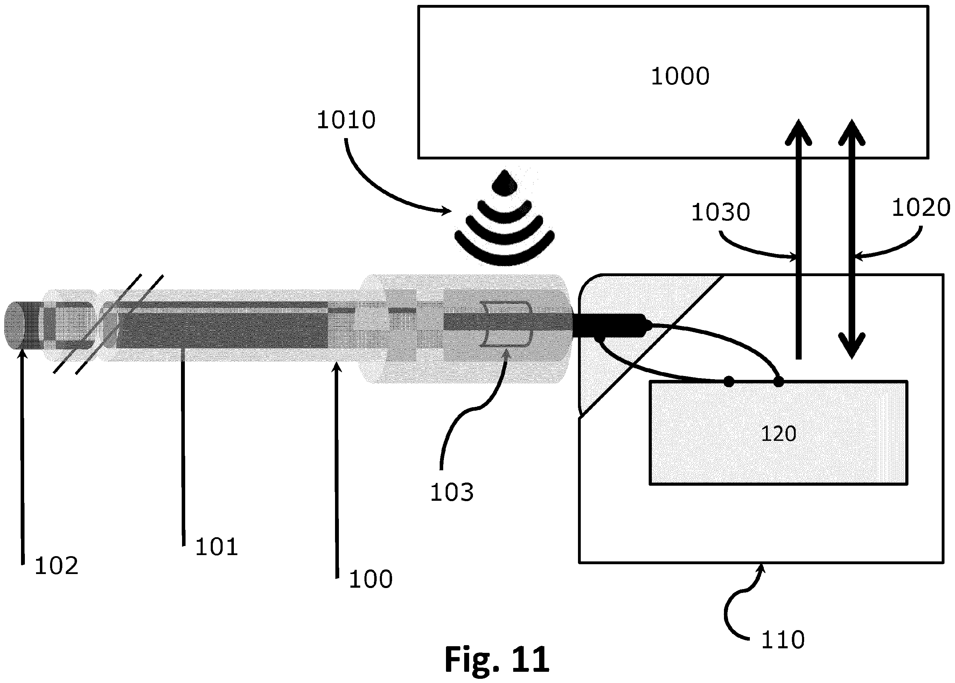

Alternativ zu der Aktivierung mittels des Implantats 110 kann, wie in Figur 11 veranschaulicht, die Aktivierung des RFID-Chips 200 des RFID-Inlays 103 auch durch ein externes Gerät 1000 erfolgen, welches ein elektromagnetisches Signal 1010 einer geeigneten Frequenz erzeugt und in die Antenne 210 des RFID-Inlays 103 einkoppelt. Auf analoge Weise wie bei der Aktivierung durch das Implantat 110 wird dann, wie oben beschrieben, durch den RFID-Chip 200 ein Antwortsignal erzeugt, welches durch die Antenne 210 des RFID-Inlays 103 in den elektrischen Leiter 101 als Antenne eingekoppelt wird. Bei diesem Ausführungsbeispiel wird vorzugsweise die Steuereinrichtung 120 des Implantats 110 durch das externe Gerät 1000 mittels einer Synchronisation 1020 aktiviert, damit die Steuereinrichtung 120 für den Empfang des durch den RFID-Chip 200 erzeugten Antwortsignals bereit ist. Die aus dem RFID-Chip 103 ausgelesenen Daten können durch eine Datenverbindung 1030 vom Implantat 110 zum externen Gerät 1000 übertragen werden.

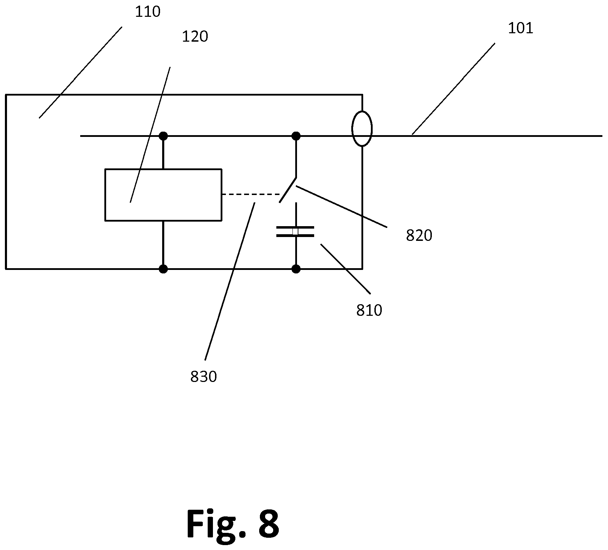

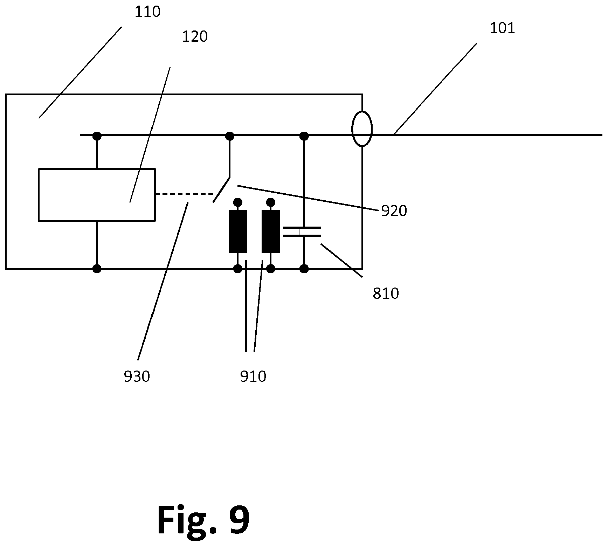

Elektronische Implantate verfügen, wie in den Figuren 8 und 9 dargestellt, häufig über EMI-Kondensatoren 810, welche hohe Frequenzen durch Ableitung auf das Implantatgehäuse herausfiltern. Insbesondere wenn die zur Identifizierung der Elektrodenleitung verwendeten Signale in diesem Frequenzbereich liegen, stört ein solcher EMI-Kondensator 810 die gewünschte Signalübertragung zum Auslesen der Elektrodenleitung(en) 100. Daher ist in dem Implantat 110 erfindungsgemäß ein Schalter 820 (siehe Figur 8) vorgesehen, der im abfragenden Teilsystem von der Steuereinrichtung 120 über eine Steuerleitung 830 betätigt wird und die Verbindung zwischen dem EMI-Kondensator 810 zur elektrischen Leitung 101 unterbricht. Dadurch werden die hochfrequenten Ströme zur Energieversorgung und Kommunikation mit dem RFID-Inlay 103 durch den EMI-Kondensator 810 nicht kurzgeschlossen. Alternativ kann die filternde Wirkung der EMI-Kondensatoren 810, wie in Figur 9 dargestellt, dadurch unterbunden werden, dass dem EMI-Kondensator 810 per Schalter 920, welcher über die Steuerleitung 930 durch die Steuereinrichtung 120 betätigt wird, jeweils eine oder mehrere geeignet dimensionierte Spulen 910 parallel geschaltet werden, so dass ein paralleler Schwingkreis mit Resonanz bei der Arbeitsfrequenz der Steuereinrichtung 120 realisiert wird. Bei Resonanz ist in diesem Fall die Impedanz des Parallelschwingkreises maximal, was einem offenen Stromkreis entspricht. Durch den Einsatz mehrerer Spulen ist der entstehende Parallelschwingkreis an verschiedene Arbeitsfrequenzen zur Energieversorgung und Kommunikation mit dem RFID-Inlay 103 anpassbar.

In einem Ausführungsbeispiel ist die Steuereinrichtung 120 des Implantats 110 derart gestaltet, dass sie in regelmäßigen oder programmierbaren Zeitabständen oder durch das Auftreten eines vorbestimmten Ereignisses das RFID-Inlay 103 der trennbar montierten Elektrodenleitungen 100 abfragt. Im Falle einer Änderung der Inhalte des Speichers des RFID-Inlays seit der letzten Abfrage wird eine Meldung generiert, die beispielsweise an eine Telemonitoring-Plattform übertragen wird und/oder bei nächster Nachsorge am Programmiergerät, das ein externes Gerät darstellt, angezeigt wird. Diese Information kann auch als Hinweis auf einen möglichen Elektrodendefekt oder einen zwischenzeitlichen Austausch einer Elektrodenleitung 100 genutzt werden.

Mit der erfindungsgemäßen Lösung können spezifische Informationen von jedem elektrischen Leiter 101 einer Elektrodenleitung 100 ausgelesen werden, ohne den therapeutischen Pfad durch eine galvanische Kopplung zu gefährden. Es ist auch keine weitere Durchführung am Implantat 110 für eine Sendeantenne notwendig, welche vorgesehen werden müsste, wenn das Antwortsignal mittels einer an dem Implantat 110 angebrachten Antenne empfangen werden würde. Von Vorteil ist außerdem, dass der Leistungsbedarf beim Auslesen eines elektrischen Leiters 101 gegenüber dem Auslesen mittels einer Antenne, die am Implantat 110 angeordnet ist, deutlich reduziert ist.

### Bezugszeichenliste

- 100: Elektrodenleitung
- 101: elektrischer Leiter
- 101a, 101b: elektrischer Leiter
- 102: Elektrodenspitze
- 103: RFID-Inlay
- 110: Implantat
- 120: Steuereinrichtung
- 200: RFID-Chip
- 210: Antenne
- 220: Induktivität zur Impedanzanpassung
- 310: Isolationsschlauch
- 320, 320a: Isolationshülse
- 330: Stecker
- 401, 402: Antenne
- 403, 404: Antenne
- 405, 406, 407: Antenne
- 408: Antenne
- 600: Keramik- oder Glashülse
- 700: Metallhülse mit Schlitz 710
- 710: Schlitz
- 810: EMI-Kondensator
- 820, 920: Schalter
- 830, 930: Steuerleitung
- 910: Spule
- 1000: externes Gerät
- 1010: elektromagnetisches Signal
- 1020: Synchronisation
- 1030: Datenverbindung

### Bezugszeichenliste

- 100: Elektrodenleitung
- 101: elektrischer Leiter
- 101a, 101b: elektrischer Leiter
- 102: Elektrodenspitze
- 103: RFID-Inlay
- 110: Implantat
- 120: Steuereinrichtung
- 200: RFID-Chip
- 210: Antenne
- 220: Induktivität zur Impedanzanpassung
- 310: Isolationsschlauch
- 320, 320a: Isolationshülse
- 330: Stecker
- 401, 402: Antenne
- 403, 404: Antenne
- 405, 406, 407: Antenne
- 408: Antenne
- 600: Keramik- oder Glashülse
- 700: Metallhülse mit Schlitz 710
- 710: Schlitz
- 810: EMI-Kondensator
- 820, 920: Schalter
- 830, 930: Steuerleitung
- 910: Spule
- 1000: externes Gerät
- 1010: elektromagnetisches Signal
- 1020: Synchronisation
- 1030: Datenverbindung

## Patentansprüche

1. Verfahren zur Identifikation einer Elektrodenleitung (100) mittels eines Implantats (110),
- wobei die Elektrodenleitung (100) einen Stecker (330), mindestens einen elektrischen Leiter (101), einen den mindestens einen elektrischen Leiter isolierenden Isolationsschlauch (310) und ein durch den Isolationsschlauch (310) oder den Stecker (330) hermetisch abgeschlossenes passives RFID-Inlay (103) mit einem RFID-Chip (200) und einer mit dem RFID-Chip (200) elektrisch leitend verbundenen Antenne (210, 401, 402, 403, 404, 405, 406. 407, 408, 700) aufweist, wobei die Antenne (210, 401, 402, 403, 404, 405, 406. 407, 408, 700) elektromagnetisch mit dem mindestens einen elektrischen Leiter (101) gekoppelt ist;
- wobei das Implantat (110) eine Steuereinrichtung (120) aufweist und das Implantat (110) mit der Elektrodenleitung (101) verbunden ist;
umfassend die folgenden Schritte:
- Senden eines elektromagnetischen Abfragesignals durch den auszulesenden elektrischen Leiter (101), wobei das Abfragesignal von der mit dem auszulesenden elektrischen Leiter (101) verbundenen Steuereinrichtung (120) oder durch ein externes Gerät erzeugt wird,
- Aktivieren des RFID-Inlays (103) durch Empfangen des Abfragesignals mittels der Antenne (210, 401, 402, 403, 404, 405, 406. 407, 408, 700) des RFID-Inlays (103) und Weiterleiten des empfangenen Abfragesignals an den RFID-Chip (200),
- Verarbeiten des empfangenen Abfragesignals durch den RFID-Chip (200), Erzeugen eines entsprechenden elektromagnetischen Antwortsignals durch den RFID-Chip (200) und Senden des Antwortsignals mittels der Antenne (210, 401, 402, 403, 404, 405, 406. 407, 408, 700) des RFID-Inlays (103),
- Empfangen des Antwortsignals durch den auszulesenden elektrischen Leiter (101) der Elektrodenleitung (100) und
- Verarbeiten des an die Steuereinrichtung (120) weitergeleiteten empfangenen Antwortsignals in der Steuereinrichtung (120)
**dadurch gekennzeichnet, dass**
während des Sendens des Abfragesignals und des Empfangens des Antwortsignals das Filtern von Signalen durch EMI-Kondensatoren (810, 910) des Implantats (110) deaktiviert wird.

2. Verfahren nach Anspruch 1, **dadurch gekennzeichnet, dass** die Erzeugung des Abfragesignals durch die Steuereinrichtung (120) in regelmäßigen und/oder vorgebbaren Zeitabständen und/oder nach dem Auftreten eines vorbestimmten Ereignisses erfolgt.

3. Implantat (110) mit einer Elektrodenleitung (100),
- wobei die Elektrodenleitung (100) einen Stecker (330), mindestens einen elektrischen Leiter (101), einen den mindestens einen elektrischen Leiter isolierenden Isolationsschlauch (310) und ein durch den Isolationsschlauch (310) oder den Stecker (330) hermetisch abgeschlossenes passives RFID-Inlay (103) mit einem RFID-Chip (200) und einer mit dem RFID-Chip (200) elektrisch leitend verbundenen Antenne (210, 401, 402, 403, 404, 405, 406. 407, 408, 700) aufweist, wobei die Antenne (210, 401, 402, 403, 404, 405, 406. 407, 408, 700) elektromagnetisch mit dem mindestens einen elektrischen Leiter (101) gekoppelt ist;
- wobei das Implantat (110) eine Buchse zur Verbindung mit dem Stecker (330) der Elektrodenleitung (100) und ein Gehäuse aufweist, wobei in dem Gehäuse eine Steuereinrichtung (120) angeordnet ist, wobei die Steuereinrichtung (120) zur Verarbeitung eines Antwortsignals eingerichtet ist, welches von dem elektrischen Leiter (101) der Elektrodenleitung (100), die mit ihrem Stecker (330) in die Buchse eingesteckt ist, so dass der elektrische Leiter (101) elektrisch mit der Steuereinrichtung (120) verbunden ist, empfangen und an die Steuereinrichtung (120) weitergeleitet wird,
**dadurch gekennzeichnet, dass**
im Implantat (110) ein Schalter (820, 920) vorgesehen ist, der die filternde Wirkung des EMI-Kondensators (810) deaktiviert,
- wobei der Schalter (820) die Verbindung zwischen einem EMI-Kondensator (810) und dem mit der Steuereinrichtung (120) verbundenen elektrischen Leiter (101) öffnet; oder
- wobei der Schalter (920) die Parallelschaltung von einer oder mehreren Spulen (910) mit einem EMI-Kondensator (810) bewirkt, so dass ein Parallelschwingkreis gebildet wird, wobei die eine oder die mehreren Spulen (910) derart dimensioniert sind, dass der Parallelschwingkreis eine Resonanz bei der Arbeitsfrequenz der Steuereinrichtung (120) aufweist.

4. Implantat nach Anspruch 3, **dadurch gekennzeichnet, dass** die Buchse eine Vielzahl von Anschlüssen zur Verbindung mit der Vielzahl elektrischer Leiter (101) einer Elektrodenleitung (100) oder mehrerer Elektrodenleitungen (100) aufweist und dass eine Sende- und/oder Empfangseinheit der Steuereinrichtung (120) vorgesehen ist, welche über einen Multiplexer mit der Vielzahl von Anschlüssen verbunden ist.

5. Implantat nach einem der Ansprüche 3 bis 4, **dadurch gekennzeichnet, dass** die Steuereinrichtung (120) derart eingerichtet ist, dass sie das Abfragesignal in regelmäßigen und/oder vorgebbaren Zeitabständen und/oder nach dem Auftreten eines vorbestimmten Ereignisses erzeugt.

## Claims

1. A method for identifying an electrode lead (100) by means of an implant (110),
- the electrode lead (100) comprising a plug (330), at least one electrical conductor (101), an insulating tube (310) insulating the at least one electrical conductor, and a passive RFID inlay (103), which is hermetically sealed by the insulating tube (310) or the plug (330) and comprises an RFID chip (200) and an antenna (210, 401, 402, 403, 404, 405, 406, 407, 408, 700) electrically conductively connected to the RFID chip (200), the antenna (210, 401, 402, 403, 404, 405, 406, 407, 408, 700) being electromagnetically coupled to the at least one electrical conductor (101);
- the implant (110) comprising a control device (120), and the implant (110) being connected to the electrode lead (101),
comprising the following steps:
- transmitting an electromagnetic polling signal by the electrical conductor (101) to be read out, the polling signal being generated by the control device (120) connected to the electrical conductor (101) to be read out or by an external device;
- activating the RFID inlay (103) by receiving the polling signal by means of the antenna (210, 401, 402, 403, 404, 405, 406, 407, 408, 700) of the RFID inlay (103) and forwarding the received polling signal to the RFID chip (200);
- processing the received polling signal by the RFID chip (200), generating a corresponding electromagnetic response signal by the RFID chip (200), and transmitting the response signal by means of the antenna (210, 401, 402, 403, 404, 405, 406, 407, 408, 700) of the RFID inlay (103);
- receiving the response signal by the electrical conductor (101) of the electrode lead (100) to be read out; and
- processing the received response signal forwarded to the control device (120) in the control device (120),
**characterized in that**
the filtering of signals by EMI capacitors (810, 910) of the implant (110) is deactivated during the transmission of the polling signal and the reception of the response signal.

2. The method according to claim 1, **characterized in that** the generation of the polling signal by the control device (120) is carried out at regular and/or predefinable time intervals and/or after the occurrence of a predetermined event.

3. An implant (110) comprising an electrode lead (100),
- the electrode lead (100) comprising a plug (330), at least one electrical conductor (101), an insulating tube (310) insulating the at least one electrical conductor, and a passive RFID inlay (103), which is hermetically sealed by the insulating tube (310) or the plug (330) and comprises an RFID chip (200) and an antenna (210, 401, 402, 403, 404, 405, 406, 407, 408, 700) electrically conductively connected to the RFID chip (200), the antenna (210, 401, 402, 403, 404, 405, 406, 407, 408, 700) being electromagnetically coupled to the at least one electrical conductor (101);
- the implant (110) comprising a socket for connection to the plug (330) of the electrode lead (100) and a housing, a control device (120) being disposed in the housing, the control device (120) being configured to process a response signal that is received by the electrical conductor (101) of the electrode lead (100), which with the plug (330) thereof is inserted into the socket so that the electrical conductor (101) is electrically connected to the control device (120), and forwarded to the control device (120),
**characterized in that**
a switch (820, 920) which deactivates the filtering action of the EMI capacitor (810) is provided in the implant (110),
- the switch (820) opening the connection between an EMI capacitor (810) and the electrical conductor (101) connected to the control device (120); or
- the switch (920) effectuating the parallel connection of one or more coils (910) with an EMI capacitor (810), so that a parallel resonant circuit is formed, the one or more coils (910) being dimensioned such that the parallel resonant circuit has a resonance at the working frequency of the control device (120).

4. The implant according to claim 3, **characterized in that** the socket comprises a plurality of ports for the connection to the plurality of electrical conductors (101) of an electrode lead (100) or plurality of electrode leads (100), and a transmitting and/or receiving unit of the control device (120) is provided, which is connected via a multiplexer to the plurality of ports.

5. The implant according to any one of claims 3 to 4, **characterized in that** the control device (120) is configured so as to generate the polling signal at regular and/or predefinable time intervals and/or after the occurrence of a predetermined event.

## Revendications

1. Procédé d'identification d'une ligne d'électrode (100) au moyen d'un implant (110),
- dans lequel la ligne d'électrode (100) présente une prise (330), au moins un conducteur électrique (101), une tuyau d'isolation (310) isolant l'au moins un conducteur électrique et une incrustation RFID (103) passive, dotée d'une puce RFID (200), fermée hermétiquement par le tuyau d'isolation (310) ou la prise (330), et une antenne (210, 401, 402, 403, 404, 405, 406, 407, 408, 700) reliée de manière électriquement conductrice avec la puce RFID (200), où l'antenne (210, 401, 402, 403, 404, 405, 406, 407, 408, 700) est couplée de manière électromagnétique avec l'au moins un conducteur électrique (101),
- dans lequel l'implant (110) présente un dispositif de commande (120) et l'implant (110) est relié avec la ligne d'électrode (101) ;
comprenant les étapes suivantes :
- l'envoi d'un signal d'interrogation électromagnétique par le conducteur électrique (101) devant être sélectionné, où le signal d'interrogation est généré par le dispositif de commande (120) relié avec le conducteur électrique (101) devant être sélectionné ou par un appareil externe,
- l'activation de l'incrustation RFID (103) par la réception du signal d'interrogation au moyen de l'antenne (210, 401, 402, 403, 404, 405, 406, 407, 408, 700) de l'incrustation RFID (103) et la transmission du signal d'interrogation reçu à la puce RFID (200),
- le traitement du signal d'interrogation reçu par la puce RFID (200), la création d'un signal de réponse électromagnétique correspondant par la puce RFID (200) et l'envoi du signal de réponse au moyen de l'antenne (210, 401, 402, 403, 404, 405, 406, 407, 408, 700) de l'incrustation RFID (103),
- la réception du signal de réponse par le conducteur électrique (101) devant être sélectionné de la ligne d'électrode (100), et
- le traitement du signal de réponse transmis reçu dans le dispositif de commande (120) vers le dispositif de commande (120),
**caractérisé en ce que**
pendant l'envoi du signal d'interrogation et la réception du signal de réponse, le filtrage de signaux par des condensateurs EMI (810, 910) de l'implant (110) est désactivé.

2. Procédé selon la revendication 1, **caractérisé en ce que** la création du signal d'interrogation a lieu à des intervalles de temps réguliers et/ou pouvant être définis, et/ou après la survenue d'un évènement prédéfini par le dispositif de commande (120).

3. Implant (110) doté d'une ligne d'électrode (100),
- dans lequel la ligne d'électrode (100) présente une prise (330), au moins un conducteur électrique (101), une tuyau d'isolation (310) isolant l'au moins un conducteur électrique, et une incrustation RFID (103) passive, dotée d'une puce RFID (200), fermée hermétiquement par le tuyau d'isolation (310) ou la prise (330), et une antenne (210, 401, 402, 403, 404, 405, 406, 407, 408, 700) reliée de manière électriquement conductrice avec la puce RFID (200), où l'antenne (210, 401, 402, 403, 404, 405, 406, 407, 408, 700) est couplée de manière électromagnétique avec l'au moins un conducteur électrique (101) ;
- dans lequel l'implant (110) présente une douille pour la connexion avec la prise (330) de la ligne d'électrode (100) et un boîtier, où un dispositif de commande (120) est disposé dans le boîtier, où le dispositif de commande (120) est prévu pour le traitement d'un signal de réponse, lequel est reçu par le conducteur électrique (101) de la ligne d'électrode (100) qui est insérée avec sa prise (330) dans la douille de sorte que le conducteur électrique (101) est relié électriquement avec le dispositif de commande (120), et est transmis au dispositif de commande (120),
**caractérisé en ce que**
dans l'implant (110), il a été prévu un commutateur (820, 920) qui désactive l'effet filtrant du condensateur EMI (810),
- où le commutateur (820) établit la connexion entre le condensateur EMI (810) et le conducteur électrique (101) relié avec le dispositif de commande (120) ; ou
- où le commutateur (920) provoque la mise en circuit parallèle d'une ou de plusieurs bobines (910) avec un condensateur EMI (810) de sorte qu'un circuit oscillant parallèle est formé, où la ou les bobines (910) sont dimensionnées de telle manière que le circuit oscillant parallèle présente une résonance à une fréquence de travail du dispositif de commande (120).

4. Implant selon la revendication 3, **caractérisé en ce que** la douille présente une multiplicité de connecteurs pour la liaison avec une multiplicité de conducteurs électriques (101) d'une ligne d'électrode (100) ou de plusieurs lignes d'électrodes (100) et qu'une unité de transmission et/ou de réception du dispositif de commande (120) est prévue, laquelle est reliée avec la multiplicité de connecteurs par le biais d'un multiplexeur.

5. Implant selon l'une des revendications 3 à 4, **caractérisé en ce que** le dispositif de commande (120) est conçu de telle manière qu'il génère le signal d'interrogation à des intervalles de temps réguliers et/ou pouvant être prédéfinis, et/ou après la survenue d'un événement prédéfini.
